# EUROPEAN PATENT SPECIFICATION

(11) **EP 4 178 528 B1**
(45) Date of publication and mention of the grant of the patent: **05.08.2026**
(21) Application number: 21730629.9
(22) Date of filing: 11.06.2021
(51) Int. Cl.: A61K 8/60, A61P 17/00, A61Q 5/00, A61Q 17/00, A61K 31/7016

(54) **USE OF TREHALOSE AS PREBIOTIC FOR INHIBITING AILMENT INDUCING MICROORGANISMS**
VERWENDUNG VON TREHALOSE ZUR HEMMUNG KRANKHEITSINDUZIERENDER MIKROORGANISMEN
UTILISATION DE TRÉHALOSE POUR INHIBER LES MICRO-ORGANISMES PROVOQUANT DES MALADIES

(30) Priority: 10.07.2020 WO PCT/CN2020/101302; 24.08.2020 EP 20192342
(43) Date of publication of application: 17.05.2023
(73) Proprietor: Unilever IP Holdings B.V., 6708 WH Wageningen (NL); Unilever Global IP Limited, Wirral, Merseyside CH62 4ZD (GB)
(72) Inventor: CHU, Chung-Ching, Shanghai, 200335 (CN); PU, Mingming, Shanghai, 200335 (CN); YIN, Qin, Shanghai, 200335 (CN); YUE, Xueyang, Shanghai, 200335 (CN)
(74) Representative: Unilever Patent Group
(86) International application number: PCT/EP2021/065735
(87) International publication number: WO 2022/008163

(56) References cited:
- WO-A1-2021/170409
- WO-A2-2019/098706
- CN-B- 103 250 880
- FR-A1- 3 064 473
- JP-A- H0 853 326
- US-A1- 2002 165 168
- US-A1- 2005 220 726
- US-A1- 2007 003 502
- DATABASE GNPD [online] MINTEL; 1 July 2020 (2020-07-01), ANONYMOUS: "Shampoo (Refresh)", XP055772551, retrieved from https://www.gnpd.com/sinatra/recordpage/7919545/ Database accession no. 7919545

## Description

### Field of the invention

The present invention relates to a novel use of trehalose. More particularly the present invention relates to a novel use of trehalose as prebiotic for promoting scalp health.

### Background of the invention

Prebiotic compositions are well known both as food compositions as well as in topical compositions. It is believed that a prebiotic composition utilized by host microorganisms (generally known as good bugs) providing a health benefit. Therefore, prebiotic compositions are using the microorganisms that anyway resides in human body either on topical surfaces or in the intestines.

Topical prebiotic composition is a subject matter of interest for both industrial and academic research. Prebiotic compositions that intended for topical application utilized by microorganism resides on the topical surface e.g. skin, scalp providing benefits associated with above mentioned surfaces.

There are prebiotic skin compositions and methods known in the art.

US2013115610 (P&G, 2013) describes a method for identifying test agents that exhibit prebiotic activity on human skin commensal microorganisms and compositions that include such agents. The method includes providing a test culture of a test agent, a human skin commensal microorganism and a minimal carbon medium. The method provides a time efficient and cost-effective way to predict in vivo prebiotic activity of a test agent on skin commensal microorganisms.

US2007190004 (Henkel, 2007) describes a method for promoting probiotic activity on the skin comprises contacting the skin with a probiotic effective amount of a substance having a probiotic effect selected from the group consisting of a plant extract, a glycerol monoalkyl ether, and a fatty acid ester or a combination of the plant extract and the glycerol monoalkyl ether whereby the growth of desired skin microbes is promoted and the growth of undesired skin microbes is inhibited.

US20050220726 (Cognis Corporation, 2005) is said to disclose use of sugar esters as active components for production of hair and skin care preparations.

There are plethora of microorganisms resides on human skin or scalp. Some of those are harmless. However, few of them may cause different illness or create some condition which are not desired by human being e.g. *Staphylococcus aureus, Malassezia restricta, Acinetobacter* etc.

One of such conditions which is not desired by people is dandruff. It is a common scalp disorder affecting many people with the symptoms of shedding, itch and visible flakes on the scalp. It has been found that in case of dandruff scalp the levels of *Malassezia restricta* (*M. restricta*) is significantly higher as compared to healthy scalp. Therefore, reducing the levels of *M. restricta* is considered by the present inventors as one of the effective approaches to effectively treat dandruff.

Therefore, a composition or material for reduction of such unwanted microorganisms is remains to be desired. Especially, a composition or material which can significantly reduce the level of *M. restricta* is a need of the present time.

### Summary of the invention

In a first aspect, the present invention provides trehalose for use as prebiotic in presence of S. *hominis* for use in a method for inhibiting ailment inducing microorganisms when applied on an external surface of a human body, wherein said microorganism is *Malassezia restricta,* and said use is for inhibiting dandruff to promote scalp health.

In a second aspect, the present invention provides a topical composition comprising trehalose for use as prebiotic in in presence of *S*. *hominis* for use in a method for inhibiting ailment inducing microorganisms when applied on an external surface of a human body, wherein said microorganism is *Malassezia restricta* and said use is for inhibiting dandruff to promote scalp heath.

These and all other aspects of the present invention will more readily become apparent upon considering the detailed description and examples which follow.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

By "A Personal Care Composition" as used herein, is meant to include a composition for topical application to the skin of mammals, especially humans. Such a composition may be generally classified as leave-on or rinse off. The composition is formulated into a product which is applied to a human body specifically for improving appearance but may also be capable of providing cleansing, odor control or general aesthetics. The composition of the present invention can be in the form of a liquid, lotion, cream, foam, scrub, gel, or toner, or applied with an implement or via a face mask or a pad. Non-limiting examples of such compositions include leave-on skin lotions, creams, antiperspirants, deodorants, lipsticks, foundations, mascara, sunless tanners and sunscreen lotions. The composition of the present invention is preferably a leave-on composition. "Skin" as used herein is meant to include skin on the face and body (e.g., neck, chest, back, arms, underarms, hands, legs, buttocks and scalp) and especially to the sun exposed parts thereof.

The term "prebiotic" with respect to the present invention means an ingredient or a composition which is used by other commensal microorganisms (good bugs) resides in the skin provides a skin benefits including inhibition of ailment causing microorganism.

There are varieties of microorganisms resides on human topical surface e.g. skin, scalp or oral surface. Not all of them are harmless. There are some microorganisms that exhibits adverse effect of the skin, scalp or oral surface.

The present invention provides ttrehalose for use as prebiotic in presence of *S*. *hominis* for use in a method for inhibiting ailment inducing microorganisms when applied on an external surface of a human body, wherein said microorganism is *Malassezia restricta,* and said use is for inhibiting dandruff to promote scalp health.

Trehalose is having the following structure:

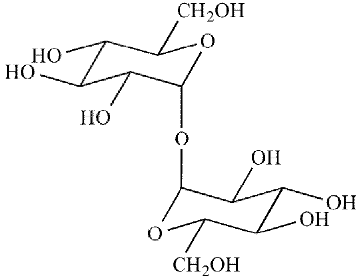

Trehalose is a disaccharide formed by a 1,1-glycosidic bond between two α-glucose units.

The present inventors have found that *S*. *hominis* present on the skin/scalp surface uses trehalose to inhibit ailment inducing microorganisms.

Hence the present invention also provides use of Trehalose in presence of *S*. *hominis* for inhibiting ailment inducing microorganisms when applied on an external surface of a human body.

Preferably, the external surface comprising of keratinocytes. Most preferably said external surface is human skin and/or scalp.

The ailment causing microorganisms are *Malassezia restricta.*

It has been found that in case of dandruff scalp the levels of *Malassezia restricta* (*M. restricta*) is significantly higher as compared to healthy scalp. We have found that trehalose is effective against *M. restricta* as prebiotic and thereby inhibits dandruff to promote scalp heath. Trehalose in presence of *S*. *hominis* inhibits *Malassezia restricta* when applied on human scalp. Therefore, the present invention also provides use of trehalose for inhibiting dandruff.

Hence the present invention also provides use of a topical composition comprising trehalose for use as prebiotic in in presence of *S*. *hominis* for use in a method for inhibiting ailment inducing microorganisms when applied on an external surface of a human body, wherein said microorganism is *Malassezia restricta* and said use is for inhibiting dandruff to promote scalp heath.

The composition preferably comprises 0.001 to 10% of trehalose by weight of the composition. More preferably the composition comprises 0.01 to 8% by weight, furthermore preferably 0.1 to 7% by weight and most preferably 0.1 to 5% by weight of trehalose.

It is preferred that the topical composition is a cosmetic composition. Alternatively, the topical composition is a medicament or a pharmaceutical composition.

The composition of the present invention can be in the form of leave-on or wash-off composition.

Leave-on composition preferably means a composition which is not required to be removed or washed off from the human body after the application of the composition. When the composition is in the form of a leave-on composition, the composition is preferably a hair cream, hair serum, deodorant (stick, roll-on or spray), a hand sanitizer, a body lotion, a skin cream or body spray. Leave-on compositions are to be distinguished from compositions which are applied to the skin and subsequently removed, either by washing, rinsing, wiping, or the like either soon after or during the application of the product. Surfactants typically used for rinse-off compositions have physico-chemical properties giving them the ability to generate foam/lather in-use with ease of rinse; they can consist of mixtures of anionic, cationic, amphoteric, and nonionic surfactants.

The topical composition is preferably in the form of emulsions, which may be oil-in-water, or water-in-oil. In some embodiments, the skin care compositions are oil-in-water emulsions. Another format is a cream, including one which has a vanishing cream base. Vanishing cream base is one which comprises 5 to 40 wt% fatty acid and 0.1 to 20 wt% soap. In some embodiments, in such creams, the fatty acid is substantially a mixture of stearic acid and palmitic acid and the soap is the potassium salt of the fatty acid mixture, although other counterions and mixtures thereof can be used. The fatty acid in vanishing cream base is often prepared using hystric acid which is substantially (generally 90 to 95 percent) a mixture of stearic acid and palmitic acid. A typical hystric acid comprises 52 to 55 percent palmitic acid and 45 to 48 percent stearic acid of the total palmitic-stearic mixture. Thus, inclusion of hystric acid and its soap to prepare the vanishing cream base is within the scope of the present invention. In some embodiments, the skin care composition comprises higher than 7 percent, or higher than 10 percent, or higher than 12 percent fatty acid.

Alternatively, the topical composition is formulated as a single use personal care towelette product.

In some embodiments, the composition is non-solid. As used herein, the term "non-solid" means that the viscosity of the compositions, e.g. as measured using a Brookfield DV-I + viscometer (20RPM, RV6, 30 seconds, 20^{s}C). In some embodiments, the viscosity is in the range of from 1 Pas to 500Pas, alternatively from 1 Pas to 200Pas, alternatively from 2Pas to 100Pas, alternatively from 3Pas to 50Pas, at 20 degrees centigrade. In some embodiments, anionic surfactants are present in the leave-on skin care composition in an amount of at most 5 percent by weight of the composition, alternatively from 0.01 percent to 4 percent by weight of the composition, alternatively from 0.01 percent to 3 percent by weight of the composition, alternatively from 0.01 percent to 2 percent by weight of the composition, alternatively substantially absent (less than 1 percent, or less than 0.1 percent, or less than 0.01 percent). In some embodiments, the total level of surfactant in the skin care compositions is no more than 10 percent, alternatively below 8 percent, alternatively at most 5 percent.

If the composition is in the form of a deodorant, the composition may preferably comprise a conventional deodorant base as the cosmetically acceptable base. By a deodorant is meant a product in the stick, roll-on, or propellant medium which is used for personal deodorant benefit e.g. application in axilla, the under-arm area, which may or may not contain anti-perspirant actives. Deodorant compositions can generally be in the form of firm solids, soft solids, gels, creams, and liquids and are dispensed using applicators appropriate to the physical characteristics of the composition. Deodorant compositions which are delivered through roll-ons generally comprise a liquid carrier.

Such liquid carrier can be hydrophobic or comprise a mixture of both hydrophilic and hydrophobic liquids. They may be in the form of an emulsion or a microemulsion. The liquid carrier or mixture of carriers often constitutes from 30 to 95 wt% and in many instances from 40 to 80 wt%. Hydrophobic liquid carriers commonly can comprise one or more materials selected within the chemical classes of siloxanes, hydrocarbons, branched aliphatic alcohols, esters and ethers that have a melting point not higher than 25°C and a boiling point of at least 100°C. Hydrophilic carrier liquids that can be employed in compositions herein commonly comprise water and/or a mono or polyhydric alcohol or water-miscible homologue. Polyhydric alcohols commonly comprise ethylene or propylene glycol, or a homologue can be employed such as diethylene glycol. Other than this suitable other vehicle and component used for deodorant composition can be added.

Wash-off composition preferably means a composition which is intended/required to be removed from the body by washing with solvent preferably water after the application of said composition like e.g. a shampoo, a hand wash composition and a face wash composition. In case of wash-off compositions, a cosmetically acceptable base preferably further comprises a surfactant.

For example, if the composition is in the form of a shampoo, in addition to water, the cosmetically acceptable base preferably comprises an anionic surfactant e.g. an alkyl sulphate and/or ethoxylated alkyl sulfate surfactant. These anionic surfactants are preferably present at a level of from 1 to 20 wt%, more preferably from 2 to 16 wt%, even more preferably from 3 to 16 wt%. Preferred alkyl sulfates are C8 to 18 alkyl sulfates, more preferably C12 to 18 alkyl sulfates, preferably in the form of a salt with a solubilizing cation such as sodium, potassium, ammonium or substituted ammonium.

It is preferred that the topical composition comprises a cosmetically acceptable base. Examples of ingredients that may be used as cosmetically acceptable base includes water, fatty acids, soaps (salts of fatty acids), alcohols and mixtures thereof.

In some embodiments, the skin care compositions of the present invention also include a cosmetically acceptable carrier. In some embodiments, where the personal care composition is a skin care composition, the cosmetically acceptable carrier is a lipophilic carrier that is liquid at temperatures up to 40°C.

The amount of the cosmetically acceptable carrier may vary in the skin care compositions according to some embodiments of the present invention. In some embodiments, the amount of the cosmetically acceptable carrier in a skin care composition may range from 1 to 99.9 percent by weight of the composition. Alternatively, the amount of the cosmetically acceptable carrier in a skin care composition may range from 70 percent to 95 percent by weight of the composition. Alternatively, the amount of the cosmetically acceptable carrier may range from 80 percent to 90 percent by weight of the composition. Alternatively, the amount of the cosmetically acceptable carrier may range from 5 to 99.9 percent by weight of the composition. Alternatively, the amount of the cosmetically acceptable carrier may range from 10 to 99.9 percent by weight of the composition. Alternatively, the amount of the cosmetically acceptable carrier may range from 15 to 99.9 percent by weight of the composition.

Cosmetically acceptable carriers suitable for the compositions include water, emollients, fatty acids, fatty alcohols, thickeners and combinations thereof.

In some embodiments, the cosmetically acceptable carrier for skin care compositions are aqueous and include water and oil emulsions of the W/O or O/W type or multiple emulsions of the W/O/W type. Water, when present in the compositions may be in amounts ranging from 5 percent to 95 percent by weight of the skin care composition. Alternatively, the water may be present in amounts ranging from 20 percent to 70 percent by weight of the skin care composition.

In some embodiments the cosmetically acceptable carrier is an emollient material. The emollient material may be in the form of silicone oils, natural or synthetic esters, hydrocarbons, alcohols and fatty acids. Amounts of the emollient material may range from 0.1 percent to 95 percent by weight of the composition.

Silicone oils may be volatile silicone oils, or non-volatile silicone oils. The term "volatile" as used herein refers to those materials which have a measurable vapor pressure at ambient temperature.

Volatile silicone oils suitable for a skin care composition according to some embodiments of the present invention may be cyclic (cyclomethicone) or linear polydimethylsiloxanes containing from 3 to 9 silicon atoms. In one embodiment, the linear polydimethylsiloxanes 5 to 6, silicon atoms.

Non-volatile silicone oils suitable for a composition include polyalkyl siloxanes, polyalkylaryl siloxanes and polyether siloxane copolymers. The non-volatile polyalkyl siloxanes useful herein include, for example, polydimethyl siloxanes with viscosities of from 5 x 10⁶ to 0.1 m²/s at 25°C. In some embodiments, the non-volatile silicone oils suitable for a skin care composition are polydimethyl siloxanes having viscosities from 1 x 10⁵ to 4 x 10⁴ m²/s at 25°C. Another class of non-volatile silicone oils suitable for a skin care composition are emulsifying and non-emulsifying silicone elastomers, such as, for example, Dimethicone/Vinyl Dimethicone Crosspolymer available as Dow Corning 9040, General Electric SFE 839, and Shin-Etsu KSG-18.

Another class of non-volatile silicone oils suitable for a skin care composition are silicone waxes such as, for example, Silwax WS-L (Dimethicone Copolyol Laurate) may also be useful.

Ester emollients may include alkyl esters of saturated fatty acids having 10 to 24 carbon atoms. Examples include, but are not limited to, behenyl neopentanoate, isononyl isonanonoate, isopropyl myristate and octyl stearate. In another example, ester emollients suitable for a skin care composition include ether-esters such as fatty acid esters of ethoxylated saturated fatty alcohols.

In another example, suitable ester emollients include polyhydric alcohol esters. Examples include, but are not limited to ethylene glycol mono and di-fatty acid esters, diethylene glycol mono- and di-fatty acid esters, polyethylene glycol (200- 6000) mono-and di-fatty acid esters, propylene glycol mono- and di-fatty acid esters, polypropylene glycol 2000 monostearate, ethoxylated propylene glycol monostearate, glyceryl mono-and di-fatty acid esters, polyglycerol poly-fatty esters, ethoxylated glyceryl monostearate, 1,3- butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of Ci -C30 alcohols. In another example, ester emollients suitable for a skin care composition according to some embodiments of the present invention include wax esters such as, for example, beeswax, spermaceti wax and tribehenin wax.

In some skin care compositions natural ester emollients are based upon mono-, di- and tri- glycerides. Representative glycerides include, but are not limited to, sunflower seed oil, cottonseed oil, borage oil, borage seed oil, primrose oil, castor and hydrogenated castor oils, rice bran oil, soybean oil, olive oil, safflower oil, shea butter, jojoba oil and combinations thereof.

Some skin care compositions may comprise suitable hydrocarbons including, but not limited to, petrolatum, mineral oil, C11 to C13 isoparaffins, polybutenes and especially isohexadecane, available commercially as Permethyl 101 A from Presperse Inc. Fatty acids having from 10 to 30 carbon atoms may also be suitable. Illustrative of this category are pelargonic, lauric, myristic, palmitic, stearic, isostearic, oleic, linoleic, linolenic, hydroxystearic and behenic acids and mixtures thereof.

In some embodiments of skin care compositions the cosmetically acceptable carrier is fatty alcohols having from 10 to 30 carbon atoms. Suitable fatty alcohols include, but are not limited to, stearyl alcohol, lauryl alcohol, myristyl alcohol, oleyl alcohol and cetyl alcohol, or mixtures thereof.

Thickeners can be utilized as part of the cosmetically acceptable carrier of skin care compositions. Typical thickeners include crosslinked acrylates (e.g. Carbopol 982(R)), hydrophobically-modified acrylates (e.g. Carbopol 1382(R)), polyacrylamides (e.g. Sepigel 305(R)), acryloylmethylpropane sulfonic acid/salt polymers and copolymers (e.g. Aristoflex HMB(R) and AVC(R)), cellulosic derivatives and natural gums. Among useful cellulosic derivatives are sodium carboxymethylcellulose, hydroxypropyl methocellulose, hydroxypropyl cellulose, hydroxyethyl cellulose, ethyl cellulose and hydroxymethyl cellulose. Natural gums suitable for skin care compositions according to the present invention include, but are not limited to, guar, xanthan, sclerotium, carrageenan, pectin and combinations of these gums. Inorganics may also be utilized as thickeners, particularly clays such as bentonites and hectorites, fumed silicas, talc, calcium carbonate and silicates such as magnesium aluminum silicate (Veegum(R)).

Amounts of the thickener may range from 0.0001 to 10 percent, alternatively from 0.001 to 5 percent.

In some embodiments, the skin care composition contains a surfactant. The total concentration of the surfactant when present may range from 0.1 percent to 90 percent, alternatively from 0.1 percent to 80 percent. The amount of surfactant is dependent on a variety of factors, including, but not limited to, the type of personal care product. The surfactant is selected from the group consisting of anionic, nonionic, cationic and amphoteric actives. In some embodiments, nonionic surfactants are those with a C₁₀-C₂₀ fatty alcohol or acid hydrophobe condensed with from 2 to 100 moles of ethylene oxide or propylene oxide per mole of hydrophobe; C2-C10 alkyl phenols condensed with from 2 to 20 moles of alkylene oxide; mono- and di-fatty acid esters of ethylene glycol; fatty acid monoglyceride; sorbitan, mono- and di- C8-C20 fatty acids; and polyoxyethylene sorbitan as well as combinations thereof. In some embodiments, the non-ionic surfactant is selected from the group consisting of alkyl polyglycosides, saccharide fatty amides (e.g. methyl gluconamides) and trialkylamine oxides.

Amphoteric surfactants suitable in skin care compositions according to some embodiments of the present invention include cocoamidopropyl betaine, C12-C20 trialkyl betaines, sodium lauroamphoacetate, and sodium laurodiamphoacetate. Anionic surfactants suitable in skin care compositions according to some embodiments of the present invention include soap, alkyl ether sulfates and sulfonates, alkyl sulfates and sulfonates, alkylbenzene sulfonates, alkyl and dialkyl sulfosuccinates, C₈-C₂o acyl isethionates, C8-C20 alkyl ether phosphates, C8-C20 sarcosinates, C8-C20 acyl lactylates, sulfoacetates and combinations thereof. Anionic surfactants are irritating to the skin, however, and skin care compositions of the invention are preferably devoid of anionic surfactants, i.e. contain less than 1 percent, and preferably less than 0.5 percent of the anionic surfactant.

In some embodiments, the skin care composition also includes 0.01 percent to 2 percent of a rheology modifier. In some embodiments, the rheology modifier is selected from the group consisting of silica such as fumed silica or hydrophilic silicas and clays such as magnesium aluminum silicate, betonites, hectorite, laponite, and mixtures thereof.

In some embodiments, the cosmetic composition, and especially a skin care composition contains sun-screen. The UV-B sunscreen oil may be selected from the class of cinnamic acid, salicylic acid, diphenyl acrylic acid, or derivatives thereof. The UV-B sunscreen oil may include one or more of octyl salicylate, 3,3,5-trimethylcyclohexyl 2-hydroxybenzoate, ethylhexyl salicylate, 2-ethylhexyl 2-cyano-3,3-diphenyl-2-propenoate, or 2-ethylhexyl- 4-methoxycinnamate (also known as octyl methoxycinnamate or "OMC"). Such UV-B sunscreen oils are typically commercially available, such as Octisalate^{™} (octyl salicylate), Homosalate^{™} (3,3,5-trimethyleyclohexyl 2-hydroxybenzoate), NeoHeliopan^{™} (a range of organic UV filters including OMC (Neo Heliopan AV^{™}) and ethylhexyl salicylate (Neo Heliopan OS^{™})), Octocrylene^{™} and Milestab 3039^{™} (2- ethylhexyl-2-cyano-3,3-diphenyl-2-propenoate) or Parsol MCX^{™} (2-ethylhexyl-4- methoxycinnamate). The amount of UV-B sunscreen oil in the personal care composition may be about 0.1 wt percent to about 20 wt percent.

The personal care composition may further include about 0.1 wt percent to about 10 wt percent of a UV-A sunscreen oil. The personal care compositions of the present technology that incorporate a UV-A sunscreen oil exhibit a significantly higher UVAPF when compared to compositions lacking the cyclocarboxylic acid. The UV-A sunscreen oil may include one or more of 4-t-butyl-4'- methoxydibenzoylmethane ("avobenzone"), 2-methyldibenzoylmethane, 4-methyl- dibenzoyl-ethane, 4-isopropyldibenzoyl-methane, 4-tert-butyldibenzoylmethane, 2,4- dimethyldibenzoylmethane, 2,5-dimethyldibenzoylmethane, 4,4'- diisopropyldibenzoylmethane, 2-methyl-5-isopropyl-4'-methoxy-dibenzoylmethane, 2- methyl-5-tert-butyl-4'-methoxy-dibenzoylmethane, 2,4-dimethyl-4'- methoxydibenzoylmethane, 2,6-dimehyl-4-tert-butyl-4'methoxydibenzoylmethane, diethylaminohydroxybenzoyl hexyl benzoate, ecamsule, or methyl anthranilate. The amount of UV-A sunscreen oil in the personal care composition may preferably be about 0.5 wt percent to about 7 wt percent, more preferably about 1 wt percent to about 5 wt percent.

The composition of any embodiment described herein preferably includes a skin lightening ingredient. Illustrative skin lightening ingredients include, but are not limited to, placental extract, lactic acid, niacinamide, arbutin, kojic acid, ferulic acid, hydroquinone, resorcinol, resorcinol derivatives (including 4-substituted resorcinols, such as especially 4-hexyl. 4-ethyl, 4-butyl, and/or 4-isopropyl resorcinols), dicarboxylic acids, 12-hydroxystearic acid ("12HSA"), and combinations of any two or more thereof. The skin lightening ingredient preferably includes a tyrosinase inhibitor to complement the melanogenesis inhibition activity of the substituted monoamines, such as kojic acid, hydroquinone and a 4-substituted resorcinol. Dicarboxylic acid skin lightening ingredients include those such as azelaic acid, sebacic acid and oxalic acid.

The amount of skin lightening ingredient may be about 0.1 wt percent to about 10 wt percent, or any range including and between these two values.

Anti-fungal agents suitable for inclusion in personal care compositions are well known to one of skill in the art. Examples include, but are not limited to, climbazole, ketoconazole, fluconazole, clotrimazole, miconazole, econazole, etaconazole, terbinafine, salts of any one or more of these (e.g., hydrochloride salts), zinc pyrithione, selenium disulfide, octopirox and combinations of any two or more thereof. Amounts of these materials may range from about 0.000001 wt percent to about 10 wt percent of the personal care composition,

The personal care composition may further include about 0.1 wt percent to about 8 wt percent of a film forming polymer. Such film-forming polymers include, but are not limited to, polyalkyleneoxy terminated polyamides (e.g., INCI name: Polyamide-3, Polyamide-4), polyether polyamides (e.g., INCI name: Polyamide-6), mixed acid terminated polyamides (e.g., INCI name: Polyamide-7), and ester terminated poly(ester-amides) (e.g., INCI name: Polyamide-8). Such film forming polymers may be synthesized or are available commercially, such as under the Sylvaclear^{™} line of products by Arizona Chemical Company, LLC and the OleoCraft^{™} line of products by Croda International PLC. Film-forming polymers also include, but are not limited to, the INCI named Polyester-5 (e.g., Eastman AQ^{™} 38S Polymer), PPG-17/IPDI/DMPA Copolymer (e.g., Avalure^{™} UR 450 Polymer), Acrylates Copolymer (e.g., Avalure^{™} AC 120 Polymer), and polysaccharides such as Xilogel (tamarin gum), lotus bean gums, tara gum, beta glucan, pullulan, carboxymethyl cellulose, hydroxypropyl cellulose, sodium alginate, potato starch, carrageenan.

Further ingredients useful in skin care compositions herein may be selected from any and all: skin conditioning agents, skin feel mildness agents, suspending agents, auxiliary thickening agents, viscosity control agents, dispersants, solubilizing/clarifying agents, stabilizers, opacifiers/pearlescent agents, chelating/sequestering agents, hydrotropes, bactericides/fungicides, antioxidants, pH control agents, buffering agents, colorants and perfumes/fragrances, water, other optional ingredients (auxiliary agents) and the like. The compositions of the present invention can also be optionally, incorporated into a water insoluble substrate for application to the skin such as in the form of a treated wipe. Preservatives can be incorporated into the personal care compositions according to some embodiments of the present invention to protect against the growth of potentially harmful microorganisms. Suitable preservatives for personal care compositions according to some embodiments of the present invention include but are not limited to alkyl esters of para-hydroxybenzoic acid. Other suitable preservatives include hydantoin derivatives, propionate salts, and a variety of quaternary ammonium compounds. Other suitable preservatives include 1,2-alkane diols (e.g. 1,2-octane diol), phenoxyethanol, methyl paraben, propyl paraben, imidazolidinyl urea, sodium dehydroacetate and benzyl alcohol.

The colorants, opacifiers or abrasives may be included at a concentration from 0.05 percent to 5 percent, alternatively between 0.1 percent and 3 percent by weight of the composition. In some embodiments, the personal care product of the present invention may also include a pepdtide, such as, for example, the commercially available pentapeptide derivative- Matrixyl^{™}, which is commercially available from Sederma, France. In another example, in some embodiments, the personal care product of the present invention may also include Carnosine.

Preferably, the composition is formulated in the form of a powder, flake, lotion, cream, gel or mousse. More preferably, the composition is formulated in the form of cream or lotion and most preferably in the form of cream. The composition is preferably of leave-on type. The packaging for the composition of this invention can be a patch, bottle, tube, roll-ball applicator, propellant driven aerosol device, squeeze container or lidded jar.

The invention will now be explained with the help of non-limiting examples.

### Examples

### Effect of trehalose in inhibiting microorganisms like Staphylococcus aureus and Malassezia restricta:

The experiments were done using the following procedure:

### Experimental procedure:

### Step 1: Microbe culture and preparation

*Staphylococcus hominis (S. hominis, ATCC* 27844), *Cutibacterium acnes (C. acnes, ATCC6919), Staphylococcus aureus (S. aureus, ATCC* 6538), *and Malassezia restricta (M. restricta,* ATCC 7877) were inoculated from freezer stock onto agar plates according to culture conditions as shown in table 1. Then, one loop of microbes from agar plate was inoculated into a liquid culture system according to the culture condition as shown in table 1. *M. restricta* were reinoculated on MLNA plate twice before use. Microbial suspension was then prepared using appropriate liquid broth as indicated in Table 1. Cell density of bacteria (*S. hominis, C*. *acnes and S*. *aureus*) was adjusted to concentration at 10⁸ cfu/mL for further use based on absorption at 600 nm. Cell density of *M. restricta* was adjusted to concentration at 10⁸ cfu/mL for further use based on absorption at 610 nm. Detailed formula of culture broth are listed in Table 2.

**Table 1**

| **Microbe Strain** | **Agar plate** | **Liquid broth** | **Temperature (°C)** |
|---|---|---|---|
| *S. hominis* | Trypticase soy agar (TSA) | Trypticase soy broth (TSB) | 37 |
| *S. aureus* | Trypticase soy agar (TSA) | Trypticase soy broth (TSB) | 37 |
| *M. restricta* | Modified Leeming and Notman Agar (MLNA) | - | 32 |

**Table 2**

| Microbe Culture Broth | Composition |
|---|---|
| Tryptic Soy Agar (TSA) | Tryptic Soy Agar (Oxoid CM0131) - 40 g |
| | DI Water to 1000 mL |
| Trypticase soy agar (TSA) containing 2% Trehalose | Tryptic Soy Agar (Oxoid CM0131) - 40 g |
| | Trehalose - 20 g |
| | DI Water to 1000 mL |
| Trypticase soy broth (TSB) | Tryptic Soy Broth (Oxoid CM0129) - 30 g |
| | DI Water to 1000 mL |
| Modified Leeming and Notman Agar (MLNA) | Bacteriological Peptone (Oxoid, LP0037) - 10 g |
| | Glucose -10 g |
| | Yeast Extract (Oxoid, LP0021) - 2 g |
| | Ox bile (Oxoid, LP0055) - 8 g |
| | Glycerol - 10 mL |
| | Glycerol Monostearate - 0.5 g |
| | Tween 60 - 5 mL |
| | Olive oil -20 mL |
| | Agar No.1 (Oxoid, LP0011) - 15g |
| | Deionized Water to 1000 mL |

### Step 2: Overlay assay

On Day 0, 15 mL of TSA containing (1) 2% Trehalose and 10⁶ cfu/ml *S. hominis ,* (2) 10⁶ cfu/ml *S. hominis* alone (without trehalose), and (3) 2% trehalose alone (without S. hominis) was solidified in a 90 cm petri dish and cultured at 37°C for four days. TSA agar without *S*, *hominis* nor trehalose was prepared in parallel as control. At the end of Day 4, 5 mL of MLNA / TSA was overlaid onto the TSA ager, and 2 µL of serially diluted target microorganism (10⁸ - 10¹ cfu/ml of *M. restricta* / *S.aureus)* was dotted onto the MLNA agar / TSA plate after solidification. The plates were further cultured according to culture conditions as shown in Table 1 to monitor the growth of the target microorganism.

At the end, Log₁₀ reduction (Kill) were determined for the targeted microorganisms, *M*. *restricta* and *S. aureus* on plate (1), (2), and (3) as compared to the respective *M*. *restricta* or *S*. *aureus* growth on control plate

**Table 3 Log₁₀ reduction (Kill) of M. restricta**

| Example No | Microorganisms | Log₁₀ reduction (Kill) |
|---|---|---|
| 1 (2% trehalose + *S. hominis*) | *M. restricta* | 5 |
| A (2% trehalose alone, without *S*. *hominis*) | *M. restricta* | 0 |
| B (*S. hominis* alone, without trehalose) | *M. restricta* | 1 |

**Table 4 Log₁₀ reduction (Kill) of S. aureus**

| Example No | Microorganisms | Log₁₀ reduction (Kill) |
|---|---|---|
| 2 (2% trehalose + *S. hominis)* | *S. aureus* | 5 |
| C (2% trehalose alone, without *S*. *hominis*) | *S. aureus* | 1 |
| D *(S. hominis* alone, without trehalose) | *S. aureus* | 2 |

From the above Table 3 and 4 it is evident that Trehalose, in the presence of S. *hominis,* is effective against the ailment inducing microorganisms like *Malassezia restricta* and *Staphylococcus aureus.*

Therefore, it is clear from the above description that trehalose can effectively be used as prebiotic for inhibiting ailment inducing microorganisms when applied on an external surface of a human body.

## Claims

1. Trehalose for use as prebiotic in presence of *S*. *hominis* for use in a method for inhibiting ailment inducing microorganisms when applied on an external surface of a human body, wherein said microorganism is *Malassezia restricta,* and said use is for inhibiting dandruff to promote scalp health.

2. A topical composition comprising trehalose for use as prebiotic in in presence of *S. hominis* for use in a method for inhibiting ailment inducing microorganisms when applied on an external surface of a human body, wherein said microorganism is *Malassezia restricta* and said use is for inhibiting dandruff to promote scalp heath.

3. The topical composition for use as claimed in claim 2 wherein the composition comprises 0.001 to 10% of trehalose.

## Patentansprüche

1. Trehalose zur Verwendung als Präbiotikum in Gegenwart von *S. hominis* zur Verwendung in einem Verfahren zur Hemmung von krankheitserregenden Mikroorganismen bei Auftragen auf eine äußere Oberfläche eines menschlichen Körpers, wobei der Mikroorganismus *Malassezia restricta* ist, und zur Verwendung bei der Hemmung von Schuppen, um die Gesundheit der Kopfhaut zu fördern.

2. Topische Zusammensetzung, die Trehalose zur Verwendung als Präbiotikum in Gegenwart von *S. hominis* umfasst, zur Verwendung in einem Verfahren zur Hemmung von krankheitserregenden Mikroorganismen bei Auftragen auf eine äußere Oberfläche eines menschlichen Körpers, wobei der Mikroorganismus *Malassezia restricta* ist, und zur Verwendung bei der Hemmung von Schuppen, um die Gesundheit der Kopfhaut zu fördern.

3. Topische Zusammensetzung zur Verwendung, wie im Anspruch 2 beansprucht, wobei die Zusammensetzung 0,001 bis 10 % Trehalose enthält.

## Revendications

1. Tréhalose destiné à être utilisé comme prébiotique en présence de *S. hominis* destiné à être utilisé dans un procédé pour inhiber les micro-organismes provoquant des maladies lorsqu'il est appliqué sur une surface externe d'un corps humain, où ledit micro-organisme est *Malassezia restricta* et ladite utilisation est pour inhiber les pellicules pour favoriser la santé du cuir chevelu.

2. Composition topique comprenant du tréhalose destinée à être utilisée comme prébiotique en présence de *S. hominis* destinée à être utilisée dans un procédé pour inhiber les micro-organismes provoquant des maladies lorsqu'elle est appliquée sur une surface externe d'un corps humain, où ledit micro-organisme est *Malassezia restricta* et ladite utilisation est pour inhiber les pellicules pour favoriser la santé du cuir chevelu.

3. Composition topique destinée à être utilisée selon la revendication 2 où la composition comprend 0,001 à 10 % de tréhalose.
